# EUROPÄISCHE PATENTSCHRIFT

(11) **EP 2 255 799 B1**
(45) Veröffentlichungstag und Bekanntmachung des Hinweises auf die Patenterteilung: **16.05.2012**
(21) Anmeldenummer: 10004550.9
(22) Anmeldetag: 30.04.2010
(51) Int. Cl.: A61K 31/23, A61K 31/231, A61K 31/232, A61K 31/355, A61K 31/593, A61K 31/047, A61K 36/185, A61P 35/00

(54) **Mittel zur Bekämpfung von Nebenwirkungen der Krebs-Chemotherapie**
Agent for combating the side effects of cancer chemotherapy
Produit destiné à lutter contre les effets secondaires de la chimiothérapie pour le cancer

(30) Priorität: 30.04.2009 DE 102009020314
(43) Veröffentlichungstag der Anmeldung: 01.12.2010
(73) Patentinhaber: Schunk, Robert, 81827 München (DE); Krug, Stefanija, 54290 Trier (DE)
(72) Erfinder: Krug, Stefanija, 54290 Trier (DE); Schunk, Robert, 81827 München (DE)
(74) Vertreter: Bernhardt, Reinhold

(56) Entgegenhaltungen:
- EP-A1- 2 201 942

## Beschreibung

Die Erfindung betrifft ein Mittel zur Bekämpfung des als Nebenwirkung der Krebs-Chemotherapie auftretenden Hand-Fuß-Syndroms, das als wesentlichen Bestandteil Hanföl enthält.

Ein solches Mittel ist aus forum Darmkrebs, Ausgabe 2-2008, Seiten 1 bis 8 bekannt. Das bekannte Mittel betrifft eine Hanföl-Salbe. Eine Hanföl-Creme ist ferner in der nachveröffentlichten EP 2 201 942 A1 beschrieben.

Bei der Chemotherapie auftretende Nebenwirkungen, wie z.B. das oben genannte Hand-Fuß-Syndrom (auch als durch Chemotherapie induziertes agrales Erythem bekannt), können das Wohlbefinden der Patienten stark beeinträchtigen. In schwerem Fall schließen feuchte Abschuppungen sowie Geschwür- und Blasenbildungen an Händen und/oder Füßen den Patienten von den üblichen Aktivitäten des Alltags aus. Bei der Chemotherapie eingesetzte Arzneistoffe, die das Hand-Fuß-Syndrom hervorrufen können, sind beispielsweise Capecitabin, Cyclophosphamid, Cytarabin, Docetaxel, Doxorubicin, 5-FU, Paclitaxel, Sorafenib und Sunitinib.

Der Erfindung liegt die Aufgabe zugrunde, bei der Krebs-Chemotherapie auftretende Nebenwirkungen zumindest abzumildern und dadurch das Spektrum der zur Chemotherapie einsetzbaren Arzneistoffe zu erweitern bzw. eine Dosisreduzierung zu verhindern und somit die Einhaltung des Therapieschemas zu gewährleisten.

Diese Aufgabe wird durch ein Mittel zur Bekämpfung des Hand-Fuß-Syndroms gelöst, das dadurch gekennzeichnet ist, dass das Mittel eine das Hanföl enthaltende, im Verdauungstrakt auflösbare Kapsel umfasst.

Durch ein solches, Hanföl enthaltendes Mittel konnte bei einer Anzahl mit Capecitabin behandelter Darm-, Magen- und Brustkrebspatienten das Auftreten des Hand-Fuß-Syndroms fast gänzlich verhindert werden, während ohne Anwendung des Mittels unter vergleichbaren Bedingungen solche Nebenwirkungen mit einer Häufigkeit von bis zu 60% auftraten.

Vorzugsweise enthält die Kapsel neben dem Hanföl einen Vitaminzusatz, bei dem es sich z.B. um das Vitamin E, das Vitamin D₃ und/oder Beta-Carotin handeln kann.

In einer Ausführungsform umfasst das Mittel eine topisch auftragbare Komponente, die zu der oral aufzunehmenden Kapsel anzuwenden ist.

Vorzugsweise umfasst die topisch auftragbare Komponente neben dem Hanföl ein weiteres Öl, insbesondere Mandel- und/oder Rosenöl. Letztere Ölbestandteile sorgen insbesondere für einen angenehmen Geruch der aufgetragenen Komponente.

Eine als Badezusatz verwendbare Komponente ermöglicht eine Behandlung der Haut von außen zusätzlich zur Behandlung durch das oral aufgenommene Mittel und ggf. die topisch auftragbare Komponente.

Vorzugsweise enthält die als Badezusatz verwendbare Komponente des Mittels Weintraubenkernpulver und ggf. einen Zusatz, der z.B. zu einer besseren Verträglichkeit des Badewassers mit der wunden Haut beiträgt.

Vorzugsweise handelt es sich bei dem Zusatz um Himalaya-Salz und/oder Natriumcarbonat.

Die Erfindung wird nachfolgend anhand von drei Ausführungsbeispielen weiter erläutert.

Einer Anzahl von 15, mit Capecitabin behandelten Darm-, Magen- und Brustkrebspatienten wurde präventiv bei Beginn der Chemotherapie und dann täglich eine Dosis von sechs, jeweils 500 mg Hanföl enthaltenden Kapseln verabreicht. Das Hanföl enthielt einen Vitaminzusatz aus Vitamin E, D₃ und Beta-Carotin.

Bei keinem der 15 Patienten ist das Hand-Fuß-Syndrom aufgetreten.

### Beispiel 2

Eine Anzahl von drei mit Capecitabin behandelter Darm-, Magen- und Brustkrebspatienten wurde nach Auftreten des Hand-Fuß-Syndroms mit sechs Kapseln, die jeweils 500 mg Hanföl sowie die unter Beispiel 1 genannten Vitaminzusätze enthielten, behandelt. Zusätzlich wurde eine Hanföl- sowie Mandel- und Rosenöl enthaltende Ölmischung topisch aufgetragen. Bei sämtlichen Patienten bildete sich das mehr oder weniger stark ausgeprägte Hand-Fuß-Syndrom innerhalb weniger Tage zurück.

### Beispiel 3

Die Behandlung erfolgte entsprechend dem Beispiel 2, wobei außerdem ein Badezusatz aus Traubenkernpulver, Himalaya-Salz und Natriumcarbonat zur Anwendung kam. Durch die Zusatzbehandlung konnte eine deutlich verstärkte Rückbildung des Hand-Fuß-Syndroms erreicht werden.

Das in allen drei Ausführungsbeispielen verwendete Hanföl enthält die in der nachfolgenden Tabelle aufgeführten Bestandteile:

| | | |
|---|---|---|
| C₄ | Buttersäure | < 0,01 g/100g |
| C₆ | Capronsäure | < 0,01 g/100g |
| C₇ | Önanthsäure | < 0,01 g/100g |
| C₈ | Caprylsäure | < 0,01 g/100g |
| C₁₀ | Caprinsäure | < 0,01 g/100g |
| C₁₂ | Laurinsäure | < 0,01 g/100g |
| C₁₄ | Myristinsäure | 0,04 g/100g |
| C_{14:1} | Myristoleinsäure | < 0,01 g/100g |
| C₁₅ | Pentadekansäure | 0,02 g/100g |
| C₁₆ | Palmitinsäure | 7,20 g/100g |
| C_{16:1} | Palmitoleinsäure | 0,14 g/100g |
| C₁₇ | Heptadekansäure | 0,05 g/100g |
| C_{17:1} | Heptadecensäure | < 0,01 g/100g |
| C₁₈ | Stearinsäure | 2,74 g/100g |
| C_{18:1} | Ölsäure | 12,81 g/100g |
| C_{18:1(tr9)} | Elaidinsäure | 0,12 g/100g |
| C_{18:1 (tr11}) | Vaccensäure | < 0,01 g/100g |
| C_{18:2} | Linolsäure | 56,20 g/100g |
| C_{18:2(tr8)} | Linolelaidinsäure | < 0,01 g/100g |
| C_{18:3} | Linolensäure | 14,60 g/100g |
| C_{18:3} | Gamma-Linolensäure | 3,21 g/100g |
| C₂₀ | Arachinsäure | 0,83 g/100g |
| C_{20:1} | Eiconsensäure | 0,61 g/100g |
| C_{20:2} | Eicosadiensäure | 0,89 g/100g |
| C_{20:2n9} | Eicosadiensäure | < 0,01 g/100g |
| C_{20:5n3} | Eicosapentaensäure | < 0,01 g/100g |
| C₂₂ | Behensäure | 0,27 g/100g |
| C_{20:6n3} | Docosahexaensäure | < 0,01 g/100g |
| C_{22:1} | Erukasäure | 0,01 g/100g |
| C₂₄ | Lignocerinsäure | 0,11 g/100g |
| C_{24:1} | Tetracosensäure | 0,03 g/100g |

## Patentansprüche

1. Mittel zur Anwendung beider Bekämpfung des als Nebenwirkung der Krebs-Chemotherapie auftretenden Hand-Fuß-Syndroms, wobei das Mittel als wesentlichen Bestandteil Hanföl enthält,
**dadurch gekennzeichnet,**
**dass** das Mittel eine das Hanföl enthaltende, im Verdauungstrakt auflösbare Kapsel umfasst.

2. Mittel zur Answendung nach Anspruch 1,
**dadurch gekennzeichnet,**
**dass** die Kapsel neben dem Hanföl einen Vitaminzusatz enthält.

3. Mittel zur Answendung nach Anspruch 1 oder 2,
**dadurch gekennzeichnet,**
**dass** die Kapsel neben dem Hanföl Vitamin E, D₃ und/oder Beta-Carotin enthält.

4. Mittel zur Answendung nach einem der Ansprüche 1 bis 3,
**dadurch gekennzeichnet,**
**dass** das Mittel eine topisch auftragbare Komponente umfasst.

5. Mittel zur Answendung nach Anspruch 4,
**dadurch gekennzeichnet,**
**dass** die topisch auftragbare Komponente neben dem Hanföl wenigstens ein weiteres Öl umfasst.

6. Mittel zur Answendung nach Anspruch 5,
**dadurch gekennzeichnet,**
**dass** als weiteres Öl Mandel- und/oder Rosenöl vorgesehen ist.

7. Mittel zur Answendung nach einem der Ansprüche 1 bis 7,
**dadurch gekennzeichnet,**
**dass** das Mittel eine als Badezusatz verwendbare Komponente umfasst.

8. Mittel zur Answendung nach Anspruch 7,
**dadurch gekennzeichnet,**
**dass** die als Badezusatz verwendbare Komponente Weintraubenkernpulver aufweist.

9. Mittel zur Answendung nach Anspruch 8,
**dadurch gekennzeichnet,**
**dass** die als Badezusatz verwendbare Komponente Weintraubenkernpulver mit einem Zusatz enthält.

10. Mittel zur Answendung nach Anspruch 9,
**dadurch gekennzeichnet,**
**dass** der Zusatz Himalaya-Salz und/oder Natriumcarbonat umfasst.

11. Verwendung von Hanföl zur Herstellung eines Mittels in Form einer in Verdaungstrakt auflösbaren, Hantöl enthaltenden Kapsel nach einem der Ansprüche 1 bis 10 zur Bekämpfung des als Nebenwirkung der Krebs-Chemotherapie auftretenden Hand-Fuß-Syndroms.

## Claims

1. A composition for use in combating hand-foot syndrome occurring as a side effect of cancer chemotherapy, wherein the composition contains hemp oil as a substantial constituent,
**characterized in that**
the composition comprises a capsule which contains the hemp oil and is dissolvable in the digestive tract.

2. The composition for use according to Claim 1,
**characterized in that**
the capsule contains not only the hemp oil but also a Vitamin addictive.

3. The composition for use according to Claim 1 or 2,
**characterized in that**
the capsule contains not only the hemp oil but also vitamin E, vitamin D₃ and/or beta-carotene.

4. The composition for use according to any of Claims 1 to 3,
**characterized in that**
the composition comprises a topically applicable component.

5. The composition for use according to Claim 4,
**characterized in that**
the topically applicable component comprises not only the hemp oil but also at least one further oil.

6. The composition for use according to Claim 5,
**characterized in that**
the further oil is almond oil and/or rose oil.

7. The composition for use according to any of Claims 1 to 7,
**characterized in that**
the composition comprises a component usable as a bath additive.

8. The composition for use according to Claim 7,
**characterized in that**
the component usable as a bath additive comprises grapeseed powder.

9. The composition for use according to Claim 8,
**characterized in that**
the component usable as a bath additive contains grapeseed power with an additive.

10. The composition for use according to Claim 9,
**characterized in that**
the addictive comprises Himalayan salt and/or sodium carbonate.

11. The use of hemp oil to produce a composition in the form of a hemp oil-containing capsule dissolvable in the digestive tract according to any of Claims 1 to 10 for combating hand-foot syndrome occurring as a side effect of cancer chemotherapy.

## Revendications

1. Agent destiné à une utilisation lors de la lutte contre le syndrome main-pied qui apparaît comme effet secondaire de la chimiothérapie du cancer, l'agent contenant, comme constituant essentiel, de l'huile de chanvre,
**caractérisé**
**en ce que** l'agent comprend une capsule contenant l'huile de chanvre, soluble dans le tractus digestif.

2. Agent destiné à une utilisation selon la revendication 1, **caractérisé en ce que** la capsule contient, outre l'huile de chanvre, un supplément vitaminé.

3. Agent destiné à une utilisation selon la revendication 1 ou 2,
**caractérisé**
**en ce que** la capsule contient, outre l'huile de chanvre, de la vitamine E, D₃ et/ou du bêta-carotène.

4. Agent destiné à une utilisation selon l'une quelconque des revendications 1 à 3, **caractérisé**
**en ce que** l'agent comprend un composant applicable par voie topique.

5. Agent destiné à une utilisation selon la revendication 4, **caractérisé en ce que** le composant applicable par voie topique contient, outre l'huile de chanvre, au moins une autre huile.

6. Agent destiné à une utilisation selon la revendication 5, **caractérisé en ce que** de l'huile d'amande et/ou de l'huile de rose est prévue comme autre huile.

7. Agent destiné à une utilisation selon l'une quelconque des revendications 1 à 7, **caractérisé**
**en ce que** l'agent comprend un composant utilisable comme adjuvant de bain.

8. Agent destiné à une utilisation selon la revendication 7, **caractérisé en ce que** le composant utilisable comme adjuvant de bain présente de la poudre de pépin de raisin.

9. Agent destiné à une utilisation selon la revendication 8, **caractérisé en ce que** le composant utilisable comme adjuvant de bain contient de la poudre de pépin de raisin avec un additif.

10. Agent destiné à une utilisation selon la revendication 9, **caractérisé en ce que** l'additif comprend du sel de l'Himalaya et/ou du carbonate de sodium.

11. Utilisation d'huile de chanvre pour la préparation d'un agent sous forme d'une capsule soluble dans le tractus digestif, contenant de l'huile de chanvre selon l'une quelconque des revendications 1 à 10 pour lutter contre le syndrome main-pied apparaissant comme effet secondaire de la chimiothérapie du cancer.
